(19)

Europäisches
Patentamt
European
Patent Office
Office européen
des brevets

(11)    EP 4 767 927 A1

(12)    **EUROPEAN PATENT APPLICATION**

(43) Date of publication:
    **01.07.2026   Bulletin 2026/27**

(21) Application number: **24461652.0**

(22) Date of filing: **27.12.2024**

(51) International Patent Classification (IPC):
    **A61B 5/0215** *(2006.01)*     **A61B 5/00** *(2006.01)*
    **A61B 5/021** *(2006.01)*

(52) Cooperative Patent Classification (CPC):
    **A61B 5/02158; A61B 5/6869; A61B 5/7225;**
    A61B 5/02141; A61B 2562/0261

(84) Designated Contracting States:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Designated Extension States:
    **BA**
    Designated Validation States:
    **GE KH MA MD TN**

(71) Applicant: **CardioCare Spolka z ograniczona
    odpowiedzialnoscia
    Spolka komandytowa
    31-159 Krakow (PL)**

(72) Inventors:
    • ZALEWSKI, Jaros aw
      32-087 Zielonki (PL)
    • SCHWARZ, Tomasz
      31-635 Kraków (PL)
    • WOJTAS, Mariusz
      34-600 Limanowa (PL)
    • GRYŇ, Karol
      25-365 Kielce (PL)

(74) Representative: **Patpol Kancelaria Patentowa Sp.
    z o.o.
    Nowoursynowska 162J
    02-776 Warszawa (PL)**

(54)    **DEVICE FOR MEASURING AND RECORDING THE SQUEEZING FORCE OF THE HEART
VENTRICLE AND METHOD FOR MEASURING AND RECORDING THE SQUEEZING FORCE**

(57)    The subject of the invention is a device (1) for measuring and recording the squeezing force of the heart ventricle exerted by the inner surface of the heart ventricle wall, which device (1) comprises a stabilizing system (2) with at least one measuring arm (3) extending therefrom, on which a sensor (9) is placed. The device (1) has a spatial design adapted to the interior of the left ventricle, allowing for multi-point contact with the endocardial surface. The subject of the invention is also a computer-implemented method of measuring and recording the squeezing force measured using a device (1) for measuring and recording the squeezing force of the heart ventricle.

Fig. 1

b

EP 4 767 927 A1

**Description**

The subject of the invention is a device for measuring and recording the squeezing force exerted by the inner surface of the heart ventricle wall and a method of measuring and recording this squeezing force.

The work of the heart involves a sequence of events associated with the rhythmic deformation of the ventricles and atria to generate a suction or pressing force, depending on the phase of systole or diastole. The pressing force of the heart ventricles is caused by their contraction, associated with a rapid reduction of the internal volume of the ventricle. As a result the blood from the ventricle is moved into the artery, which in the case of the right ventricle is the pulmonary artery, and in the case of the left ventricle, the aorta. During this time, the atria passively fill with venous blood, and at the end of the diastolic phase they actively supplement the volume of the dilated ventricles, thus completing the cardiac cycle. From a mechanical point of view, ventricular systole is divided into two phases: isovolumetric and isotonic ejection, while diastole consists of four phases: isovolumetric diastole, early filling, diastase, and atrial filling.

Under physiological conditions, the load on the ventricular muscle is submaximal, which is associated with the fact that only a part of the range of sarcomere shortening (action units of striated muscle) is used to generate tension that balances the load resulting from the blood volume, and the remaining range of sarcomere shortening is used to perform external work. The left ventricle, pumping blood into the aorta and vessels, performs a mixed isotonic contraction with secondary loading. The more of the sarcomere shortening range is used for ejection and the less for myocardium tension, the more favourable the hemodynamic and energetic conditions are in the muscle.

In isometric contraction, the measure of the contractile capacity is the maximum tension generated by the muscle, which depends on the resting stretch of the ventricle, i.e. preload. In turn, the measure of isotonic contraction is the shortening velocity, which depends on the afterload.

The pressure in the heart cavities exerts a force on the walls of the ventricles that is balanced by the wall tension. If the wall is thick, the tensile stress is distributed over a larger number of cardiomyocytes (myocardium cells); if it is thinner, the stress is distributed over a smaller number of cardiomyocytes. The measure of the stretch of an individual cardiomyocyte is the wall stress.

The mechanical function of the myocardium is inextricably linked to the generation of electrical impulses. For this reason, one of the methods used to diagnose heart function is to measure changes in electrical potential. The most popular diagnostic technique based on electrical phenomena occurring in the heart is electrocardiography, in which the heart cycle is described by a graph composed of waves illustrating changes in electrical potential. Another popular method - echocardiography, is associated with the determination of the mechanical and flow function of the heart using ultrasound techniques. In the clinical settings, circular stress is determined based on echocardiographic measurement of the left ventricular short axis radius and ventricular wall thickness. The load on cardiomyocytes in the wall increases with increasing blood pressure and ventricular radius and is greater the thinner the ventricular wall. Stress is well correlated with oxygen consumption per gram of myocardium, indicating that it is a good measure of load on individual cardiomyocyte. For practical reasons it is estimated only at the beginning and at the end of the isovolumetric contraction. However, there is no data on the direct measurement of mechanical stress and tension of the heart ventricular walls, especially in cardiac dysfunction, for example during myocardial infarction.

Exerting a squeezing force on the blood-filled inside the heart ventricles to effectively move it into the arteries with a pressure that ensures proper filling of the entire system is a very important element of the heart's work. An indirect method allowing determination of correctness in this respect is the measurement of arterial blood pressure in two ranges, i.e. the so-called systolic pressure, which is physiologically approximately 120 mm of mercury in an adult, and the diastolic pressure with an average value of approximately 80 mm of mercury. This measurement is performed at some distance from the heart (usually on the brachial artery), which introduces numerous confounding factors. One such factor is the resistance of the arterial walls to the flowing blood. Therefore, measurement of arterial blood pressure does not reflect sufficiently the actual pressing force of the myocardium and the distribution of these forces within the heart ventricles.

Given the very complex nature of ventricular contraction, performing such a measurement is very difficult. The heart ventricle during systole not only linearly decreases its volume, but also completely changes its external and internal geometry through the torsional movement of the ventricular wall muscle. This type of contraction allows for more efficient use of the energy stored in the cells, generating maximum pressure force with minimal effort, thanks to the use of additional energy associated with the change in geometry involving torsion of the heart ventricle in the long axis. The process of evolution of the heart has led to the adaptation of its anatomical and histological structure and physiological and motor function to the most efficient pumping of blood with the maximum possible reduction of effort and energy expenditure to maintain its continuous and long-term work. However, the solutions that nature has developed for the efficiency and durability of the myocardium have become a factor that significantly complicates the diagnostics of some elements of its function. It should be assumed that the force exerted by some areas of the inner surface of ventricular wall will be greater than others, and greater than the average for the entire surface. This unequal distribution of forces could consequently cause unequal loading of the myocardium fibers and faster "wear and tear" of some of its areas, but in the process of evolutionary adaptation they were appropriately strengthened. It seems that using an appropriate multi-point measurement system, it would be possible to determine such diverse areas and the pattern of force distribution on the inner surface of the heart ventricle walls. Based on such a developed model, it would be possible to expand the existing diagnostic possibilities by considering deviations from the normal distribution of forces inside the working heart ventricle, which would potentially allow to designate areas of increased risk of weakening or hypertrophy of muscle fibers, or impairment of their motor functions.

In the invention, to determine the value of the squeezing force generated by the ventricle wall during contraction, dynamometers known in mechanics are used. These are elements equipped with special sensors that record the values of deformations caused by the force that is to be measured. If the deformation is elastic, the force is calculated using Hooke's law

$$\sigma = \varepsilon * E,$$

where $\sigma$ is the stress defined as the force F acting on the surface S; $\varepsilon$ is the deformation defined as the change in geometry, for example, in the case of one-dimensional deformation, it is the change in length $\Delta l$; E is the Young's modulus (material constant).

Therefore, the required force is

$$F = (\varepsilon * E)/S.$$

There are several methods that can be used to measure deformation, but the most popular one is based on electrical resistance tensometry, where the deformations of the measuring element $\varepsilon$ causing changes in its geometry $\Delta l$ result in a change in its electrical resistance $\Delta R$. This relationship is described by the equation

$$\Delta R/R = k * \varepsilon,$$

where k is the strain gauge constant. Therefore, by recording resistance changes, the deformation value can be determined and consequently the stress value and thus the force causing this deformation.

In the prior art, for example from patent EP 0 473 070 B1, there is known a system for myocardial tensometry built into an implantable electrotherapy apparatus for measuring myocardial contractions. The system includes a single strain gauge element positioned at a location that is subject to bending due to cardiac contractions, wherein the strain gauge element comprises a piezoelectric material or a variably resistive material, and mechanical stresses to which the strain gauge element is subjected produce in the element a voltage or resistivity changes comparable in frequency and amplitude to the cardiac contractions.

Another known solution from the prior art is the invention described in patent US 6 600 948 B2. The object of the invention is to enable the simultaneous measurement of physiological signals by multiple sensors within the human body, for example in the heart, while providing accurate measurement of at least the relative locations of all sensors, thereby enabling mapping of the electrical activation time in the myocardium. In one aspect of the invention, sensors are attached to the catheter, and more specifically, a multiple sensors/electrodes are attached to the distal end of the catheter that is inserted into the heart. In one embodiment, the sensor is a strain gauge useful for determination of myocardial contraction.

The prior art lacks solutions that provide measurement of the actual squeezing force of the heart ventricles and the distribution of forces within the inner surface of the ventricular walls, and such information would provide precise information about the properties of the myocardial function through direct measurement, which could significantly increase the range of diagnostic possibilities for both scientific purposes associated with the research of anatomical and physiological properties of the heart, as well as with a medical practice involving the diagnosis, prevention and treatment of heart dysfunctions, which are the most common cause of death.

Considering the existing deficiencies in the prior art, the Applicant has developed a solution in accordance with the attached independent claims 1 and 16, which allows for determining the above-described forces and their distributions. According to this solution, the Applicant has developed a device for measuring and recording the squeezing force of the heart ventricle, enabling collection of these measurements, and a method of measuring and recording the squeezing force of the heart ventricle. Possible embodiments of the device are presented in dependent claims 2 to 15. The device can be inserted into the appropriate heart ventricle to monitor heart work by measuring and recording the squeezing force, and the method allows for monitoring, recording, and processing measurements from the device.

A device for measuring and recording the squeezing force exerted by the inner surface of the heart ventricle wall, characterized in that the device comprises a stabilizing system with at least one flexible measuring arm extending therefrom, wherein the proximal end of the measuring arm is connected with the stabilizing system and the distal end rests on the endocardium of the left ventricle wall, wherein the device has a spatial structure adapted to the interior of the left ventricle, allowing for multi-point contact with the endocardium surface, wherein the flexible measuring arm deforms with the contraction and diastole of the ventricle and is provided with a sensor for measuring the deformation of the measuring arm reflecting the squeezing force of the heart wall on the interior of the ventricle.

Preferably, the device for measuring and recording the squeezing force of the heart ventricle comprises a stabilizing system for maintaining a stable position of the device in the left ventricle, wherein the stabilizing system comprises a stabilizer and a core extending centrally in the long axis of the left ventricle with one end in the apex of the heart and the other end in the aortic valve region.

Preferably, the sensor is a strain gauge sensor.

Preferably, the flexible measuring arms attached to the core of the stabilizing system are arranged radially relative to the long axis of the left ventricle, at equal angular distances from each other, on at least two levels, the lengths of the measuring arms being adapted to the level and ensuring adherence to the periapical and middle segments of the left ventricle.

Preferably, the point of contact of the flexible measuring arm with the endocardium is atraumatic, ensuring deformation of the measuring arm during contraction while not risking damage to the endocardium.

Preferably, the measuring arm ends with one of the following: a loop, a ball, or a spherical mesh.

Preferably, the sensor located on the measuring arm is positioned as close as possible to the proximal end of the measuring arm.

Preferably, the flexible measuring arm is made of a material that allows it to bend under the influence of contraction and to unbend it to a resting position during diastole of the left ventricle, without disturbing the natural function of the left ventricle, wherein the measuring arm is made of a material and/or comprises at least one marker that allows its visualization.

Preferably, each flexible measuring arm has dimensions allowing the sensor to be installed on it.

Preferably, the stabilization system also comprises a stabilizing guide ending with a curve at the apex of the heart, preferably the curve ending the stabilizing guide is J-shaped, and the stabilizer of the stabilization system is an O-shaped loop.

Preferably, the sensor is connected to its own resistor system forming a Wheatstone bridge, which together with the voltmeter forms a measuring system generating an electric signal corresponding to the deflection force of the measuring arm, the measuring system being connected to a microcontroller and the data collected by the sensors being transmitted to the microcontroller.

Preferably, elements transmitting the measurement signal from the individual measurement systems to the microcontroller are placed along the core located centrally in the long axis of the left ventricle.

Preferably, the microcontroller has a number of active channels enabling the simultaneous collection of a signal from all measuring systems of the device and receives at least one additional supplementary signal from other diagnostic equipment.

Preferably, each flexible measuring arm indicates a single point value of the squeezing force exerted by the heart ventricle wall on its interior.

Preferably, the flexible measuring arms and the elements of the stabilizing system are foldable and fit into a catheter that is inserted into the heart, wherein the measuring arms and the elements of the stabilizing system assume the measuring position after the catheter is slid off them in the left ventricle, then the distal ends of the measuring arms rest on the endocardium of the left ventricle wall, and after the measurement is completed, the arms and the elements of the stabilizing system are folded and hidden in the catheter that is slid onto them and then removed from the heart ventricle to the outside.

A computer-implemented method of measuring and recording the squeezing force of a heart ventricle using a device for measuring and recording the squeezing force of a heart ventricle according to the invention, characterized in that in the absence of squeezing force, the sensor remains in a rest position and its measuring system indicates a voltmeter value of 0; when a squeezing force is applied, the sensor is deformed and its measuring system indicates a voltmeter value other than 0, wherein this value is proportional to the resistance R of the sensor, which allows for determining the squeezing force exerted by a specific area of the heart ventricular wall with which the measuring arm of the device is in contact during contraction and diastole, by converting the recorded voltage into the value of the squeezing force.

Definitions of terms used in the description, which will facilitate the understanding of the structure and functionality of the device, are presented in Table 1 below.

Table 1

| Atraumatic | Not creating a risk of tissue damage, here endocardium |
|---|---|
| Level | A location along the length of the device core at some distance from the beginning or end of the core |
| Visualization | Observing a device or element placed in an organ |

(continued)

| Visualization marker | A marker that enables the observation of the device or its element using X-ray/ultrasound or other imaging techniques, to enable the observation of the device in the heart ventricle, allowing precise insertion and positioning of the device in the left ventricle |
|---|---|

An exemplary implementation of the device for measuring and recording the squeezing force of the heart ventricle according to the invention is shown on the figures, wherein this implementation and its presentation are intended to facilitate the understanding of the structure and operation of the device for measuring and recording the squeezing force of the heart ventricle, however, it is not the only possible implementation of the present invention. On the figures:

fig. 1a shows a device for measuring and recording the squeezing force of the heart ventricle in an unfolded position according to an embodiment of the invention;
fig. 1b shows a device for measuring and recording the squeezing force of the heart ventricle from fig. 1a, placed in the heart during the diastolic phase;
fig. 1c shows a device for measuring and recording the squeezing force of the heart ventricle from fig. 1a, placed in the heart during cardiac contraction;
fig. 2a shows the device for measuring and recording the squeezing force of the heart ventricle from fig. 1a, in a top view;
fig. 2b shows a top view of a device for measuring and recording the squeezing force of the heart ventricle according to an embodiment of the invention, in which the arms on the levels are not arranged identically;
fig. 3a shows the device for measuring and recording the squeezing force of the heart ventricle from fig. 1a with the individual components marked;
fig. 3b shows a view of the arm of the device for measuring and recording the squeezing force of the heart ventricle from fig. 1a;
fig. 4a shows the arm of the device with a strain gauge sensor of the initial length attached to the core;
fig. 4b shows the arm of the device deforming under the action of a squeezing force;
fig. 4c shows a comparison of the strain gauge length before and during the application of a squeezing force;
fig. 5 shows the structure of the measuring system containing a sensor for measuring deformation.

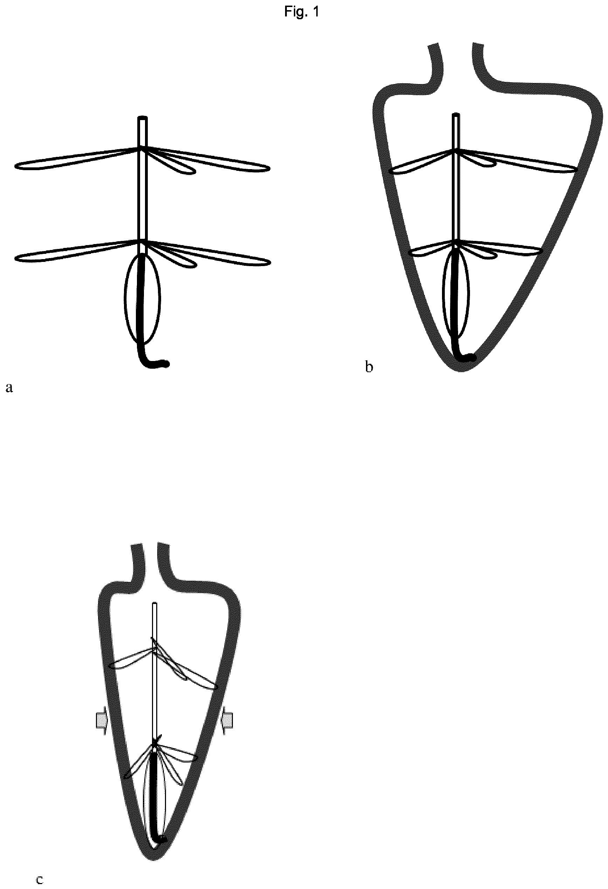

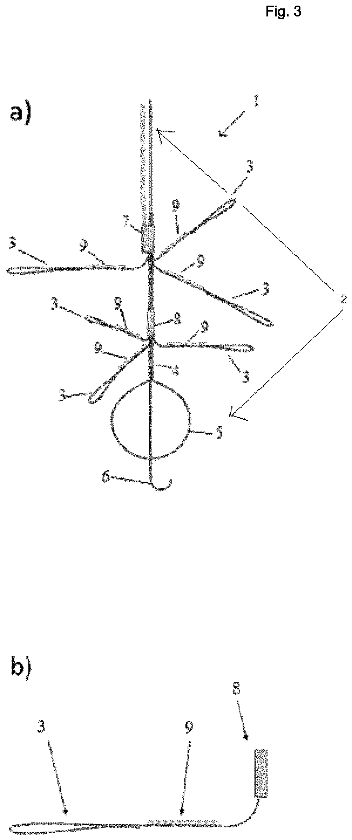

The device 1 for measuring and recording the squeezing force exerted by the inner surface of the heart ventricle wall, according to the embodiment shown in fig. 1a, 1b and fig. 1c and in fig. 3a and 3b, comprises a stabilizing system 2 from which flexible measuring arms 3 extend, wherein the proximal end of each arm 3 is connected to the stabilizing system 2 intended to stabilize the position of the device in the left ventricle, and the distal end of each arm 3 rests on the endocardium of the left ventricle wall of the heart. Each arm 3 is provided with a sensor to measure the deformation of that arm 3, reflecting the squeezing force of the heart wall on the interior of the ventricle. The design of the device 1 is spatial such that it fits inside the left ventricle while allowing for multi-point contact with the endocardial surface, as shown in fig. 1b. The design of the device 1 for measuring and recording the squeezing force of the heart ventricle when placed in the left ventricle and in its target position has the shape of an upside-down Christmas tree.

The stabilizing system 2 comprises a guide 6, a stabilizer 5, and a core 4 which is in the form of a hollow tube that extends centrally in the long axis of the left ventricle, from the left ventricular outflow tract to the cardiac apex. In other words, one end of core 4 is located in the left ventricle beneath the aortic valve and this end may be called the "upper" or "proximal" end of core 4, and the other end of core 4 is located near the apex of the heart and may be called the "lower" or "distal" end. The core 4 is straight and preferably rigid and adapted to receive, in its hollow center, a guide 6 which is attached to the proximal part of the core 4 by means of a sleeve 7 securing the guide 6. Furthermore, in the exemplary embodiment the guide 6 is stabilized inside the core 4 by clamping the core 4 thereon in the spaces between the levels. The guide 6 extends axially along the entire length of the core 4 and exits the core 4 at its distal part, where it ends in a curve that bends upwards, extends beyond the core 4 and extends below the lower edge of the stabilizer 5, and also exits the core 4 at its proximal part and extends for a certain distance. In one embodiment, the guide 6 consists of two separate, unconnected wires: a guiding one and a stabilizing one. In the distal part of the core 4, the stabilizing wire protrudes beyond the core 4 and forms the distal part of the guide 6, ending with the curve described above and positioned at the apex of the left ventricle. In this embodiment, the guide 6 is fixed in the core 4 as follows: a stabilizing wire of the guide 6 is inserted into the core 4 provided with arms 3 so that its distal end protrudes from the distal end of the core 4 as described above, while its proximal end protrudes beyond the upper end of the core 4. Then the core 4 is clamped onto this stabilizing wire between the lower levels of the arms 3 and then the guiding wire is inserted up to this clamping so that it protrudes beyond the core 4 in the proximal part of the core 4 and additionally the guiding wire is fixed in the proximal part of the core 4 with the sleeve 7. In this embodiment, the guiding wire of the guide 6 does not extend as far as the distal portion of the core 4 but only to a portion of the length of the core 4, for example to the portion of the core between the levels of the arms 3. In the proximal part of the core 4, both wires constituting the guide 6 are placed in a common biocompatible protective sheath.

The stabilizer 5 is in the form of a loop used to stabilize the position of the device 1 in the heart - together with the arms 3 it prevents unwanted movement and displacement of the device 1 in the heart ventricle, wherein if the stabilizer 5 and the curvature of the guide 6 are considered as two-dimensional elements (having no thickness), then the stabilizer loop 5 extends in a plane other than the curvature of the guide 6, for example, if the curvature of the guide 6 extends in the XZ plane, where Z is an axis coinciding with the axis of the core 3 in the Cartesian coordinate system, and the other axes are perpendicular to it, then the stabilizer loop 5 may extend, for example, in the YZ plane (the angle between the plane of curvature of the guide 3 and the stabilizing loop will then be 90 degrees).

The curve ending the guide 6 may have different shapes, for example, as shown in figs. 1a, 1b and 3a, it may be J-shaped.

Alternatively, the curve ending the guide 6 may be O-shaped, which may surround the lower part of the stabilizing loop, thereby providing even greater stabilization of the device 1.

In still alternative embodiments, the curve ending the guide 6 may have a different shape, as long as it is atraumatic, i.e., does not pose a risk of endocardial damage.

Flexible measuring arms 3 of the device 1 extend from the stabilizing system 2, or more precisely from the core 4 of this system, with the proximal end of arm 3 being located closer to and the distal end of arm 3 being located further away from the core 4.

The flexible measuring arms 3 attached to the core 4 of the stabilizing system 2 are arranged radially relative to the long axis of the left ventricle, in one embodiment, they are arranged at equal angular distances from each other at two different levels, i.e. at different places along the length of the core 4 - the distances from the upper end of the core 4 of each of the levels are different. It can be assumed that the core 4 is divided into several sections in a vertical arrangement, and each section has several arms 3 arranged concentrically in a star arrangement.

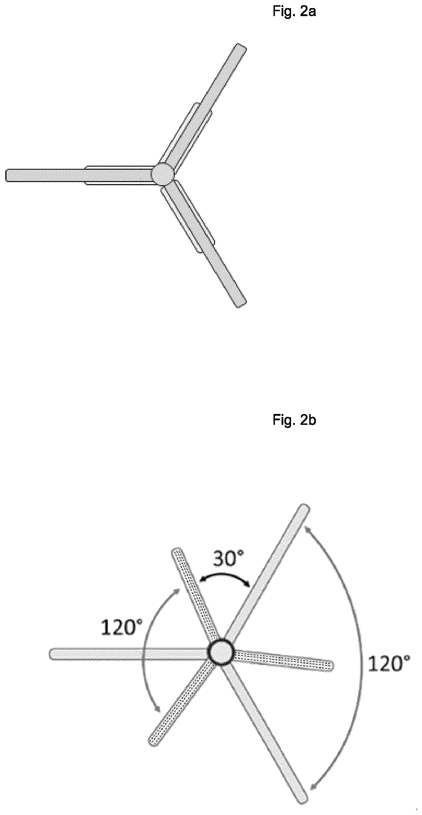

According to the embodiment shown in figs. 1a, 1b and 1c, the device 1 comprises 6 flexible measuring arms 3 on two levels, which results in 3 arms on each level. The arrangement of arms 3 at each of the levels may be identical, as shown in fig. 2a, may differ or be the same in terms of the spacing of arms 3 (their angular distances between each other), but may differ in terms of the position of arms 3 in the XZ plane, where Z is the axis coinciding with the axis of the core 4 in the Cartesian coordinate system, i.e. when looking at the device 1 "from above" from the upper end of the core 4, "downwards", the arms 3 at the individual levels do not "overlap" but are shifted relative to each other, as shown in fig. 2b.

In an alternative embodiment, the arms 3 may be arranged at more than 2 levels, and their number per level may vary and may be arbitrary, but preferably the device 1 comprises at least two flexible measuring arms 3 fixed on at least 2 levels, to provide at least two different points of contact with the endocardium and thus at least two measuring points.

It is possible to make a device 1 containing one flexible measuring arm 3, but it will not provide a satisfactory number of measurements. The number of levels depends on the size of the left ventricle of the examined heart, which is described in more detail below.

For example, the device may comprise 5 arms arranged on 2 levels, or 9 arms arranged on 3 levels, or any other feasible combination.

The lengths of the flexible measuring arms 3 are adapted to the dimensions of the level at which they are located and to the geometry of the heart ventricle so that they ensure adhesion of the flexible measuring arms 3 to the periapical and middle segments of the left heart ventricle, that is, they provide contact points of the measuring arms 3 with the endocardium, the width of the flexible measuring arms 3 ensures free placement of the sensor 9 on them and their thickness is defined by the thickness of the material from which the arms 3 are made.

Each point of contact of each flexible measuring arm 3 with the endocardium is atraumatic, i.e. there is no risk of damage to the endocardium when the measuring arm 3 is deformed during cardiac contraction. As shown in figs. 1a and 1b and also in fig. 3b such an ending has the form of a loop, however, alternatively, the flexible measuring arm 3 may be ended with a spherical mesh and/or a ball, but on the basis of the tests carried out it turned out that ending the measuring arm 3 with a loop is the most advantageous.

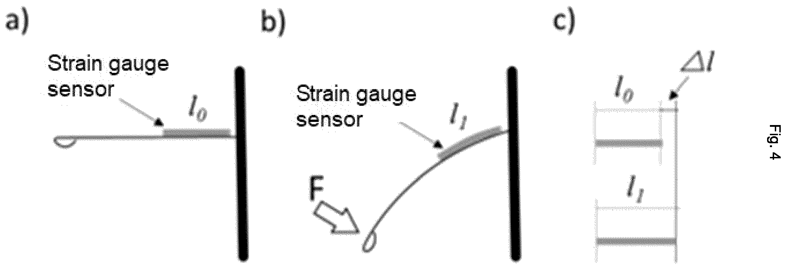

Each measuring arm 3 is deformed with the systole and diastole of the heart ventricle, in other words, during the contraction of the heart ventricle, a squeezing force F acts on the measuring arm 3 causing it to bend, which changes the length of the sensor 9 from the initial length $l_0$ to the length $l_1$, which differs from the length $l_0$ by $\Delta l$, as shown, for example, in figs. 4a, 4b and 4c. Each arm 3 of the device 1 is curved at its proximal end so that the proximal end of the arm 3 itself extends substantially parallel to the core 4, which facilitates attachment of the arms 3 to the core 4 by means of the sleeves 8 for attaching the arms 3. In the example shown in figs. 3a and 3b, the distal end of the arm 3 is ended with a loop which rests on the heart wall, and a sensor 9 is placed on the upper or lower surface of each arm 3. In the embodiment in which the sensor 9 is arranged on the lower side of the arm 3, the deformation value obtained is the same, only with a negative sign, the value itself being important for the reading of the squeezing force. In the embodiment examples, the sensor 9 is a strain gauge sensor that allows to measure the deformation of the measuring arm 3, reflecting the squeezing force of the heart wall on the interior of the ventricle, wherein the strain gauge sensor 9 is located on the upper part of the arm 3 not covered by the mounting sleeve 8, as close as possible to the core 4. A preferred embodiment of the arm 3 and the arrangement of the strain gauge sensor 9 is shown in figs. 3a and 3b.

The flexible measuring arms 3 can be of different lengths at different levels to allow the device 1 to better fit the shape of the left ventricle. In general, the dimensions of the human left ventricle in short axis during diastole at the base of the ventricle range from 38 to 60 mm in healthy individuals. In people after a heart attack, the dimensions of the ventricle are individually and variably enlarged, in extreme cases reaching 70 to 80 mm in the short axis at the base. In healthy individuals, the long axis dimensions of the left ventricle in diastole range from 50 to 100 mm, depending on gender and age. Therefore, the size of the device 1 according to the invention must be adapted to the size of the left ventricle for which it is intended. In other words, the device 1 according to the invention is manufactured in several sizes, which makes it possible to select a suitable size for individual heart dimensions.

The dimensions of the device 1 for measuring and recording the squeezing force of the heart ventricle are no more than 100 mm in the long axis in the heart ventricle (total length of the core 4, the stabilizing loop 5 and the part of the guide 6 protruding beyond the core 4 in its distal part, not including the length of the guide 6 protruding from the core 4 from its proximal part beyond the heart ventricle), and no more than 65 mm in the short axis (axis perpendicular to the long axis) for the most proximal level, i.e. the level closest to the base of the heart. The length of the single flexible measuring arm 3 can range from 10 to 45 mm. The core 4 may have a length of 35 to 45 mm and an outer diameter of 0.50 to 1.00 mm and a wall thickness of 0.1 to 0.2 mm. The stabilizer 5 may have a loop diameter of 25 mm, while the part of the guide 6 protruding beyond the lower edge of the loop of the stabilizer 5 extends below the loop by 5 to 10 mm.

From the proximal side of the core 4, the length of the guide 6 protruding beyond this core 4 depends on the point of introduction of the device 1 into the body. For carotid artery access, the length of guide 6 will be shorter than for femoral artery access. In principle, the guide 6, in the part extending from the proximal part of the core 4 to the insertion point, must have a length such that it protrudes beyond the (cardiac) catheter introducing the device 1, which means that with a catheter of 1200 mm in length, the total length of the guide 6 is approximately 1600 mm, for example 1500 mm.

Typically, considering the average statistical dimensions of the human heart ventricle, the number of levels with arms 3 will be two or three, and the preferred number of arms 3 on a single level is three to five. The distance between the levels at two levels will be 30 to 40 mm, and the distance of the level closer to the apex of the heart from that apex of the heart will also be about 30-40 mm. The implementation of the device 1 with an asymmetric arrangement of arms 3, i.e. with an odd number of arms 3 on one level, is advantageous due to the need to prevent mutual influence of the measuring arms 3.

Basically, in use, the number of levels of the device 1 on which the flexible measuring arms 3 with the strain gauges 9 are arranged, as well as the number of arms 3 on a single level, is selected depending on the size of the ventricle cavity. In other words, the person selecting the appropriate device 1 for measuring and recording squeezing force will select from the available sizes the device 1 that will provide the best fit to the given left ventricle - it will provide the contact points described above, and the left ventricular size will be determined during the ultrasound examination or any other appropriate imaging test.

The longitudinal dimension of the flexible measuring arms 3 of the device 1 for measuring and recording the squeezing force may be, for example, from 25 to 35 mm at the lower level and from 30 to 35 mm at the upper level. The transverse dimension of the arms 3 must allow for the installation of a sensor 9 on each of them, as standard, the width of the arm is 0.814 mm and its thickness is 0.153 mm. In the embodiment with three arms 3 per level, the angular distance between the arms 3 is 120 degrees. The proximal end of each arm 3, which is perpendicular to the core 4 due to the bend ensuring its adhesion to the core, is 5 mm and is covered by a sleeve 8.

As mentioned above, a sensor 9 is provided on each flexible measuring arm 3, with the sensor being as close as possible to the proximal end of the measuring arm 3 to ensure that it does not interfere with the deformation of the flexible measuring arm 3 upon contraction to provide the most accurate measurements.

Each sensor 9, such as a strain gauge, of the device 1 is connected to its own resistor system forming a Wheatstone bridge, which together with a voltmeter forms a measuring system generating an electrical signal corresponding to the deflection force of the measuring arm 3. In the embodiment shown in fig. 3a, one strain gauge sensor 9 is placed on each of the 6 flexible measuring arms 3, and the rest of the measuring system for each arm is located outside the patient, the measuring system being connected to a microcontroller located outside the patient, and the data collected by the strain gauge sensors 9 are transmitted to this microcontroller by means of elements transmitting a measuring signal, in particular wires capable of transmitting a signal, such as a copper wire with a diameter of 0.04 mm.

The wires transmitting the signals are combined into a common bundle of wires extending along the core 4 of the device 1, on its exterior, from the strain gauges 9 upwards, to lead the bundle out of the left ventricle, through the peripheral artery and out of the body to a male VGA plug and connecting, via a system of suitable amplifiers and transducers, to a microcontroller located outside the patient and equipped with a female VGA plug. In one variant, the microcontroller is connected to wires collecting a signal synchronized in time with that flowing from the strain gauge sensors 9, for example from the ECG from the patient's surface, constituting an objective reference system for the interpretation of the strain gauge measurement results.

In an alternative embodiment, the wires transmitting the signals are inserted into the core 4 and run along the core 4 upwards within the core and then are routed away from the patient, either together or bundled into a single common wire analogously to the embodiment described above.

Each wire or bundle of wires is covered with a shield, preferably a thin-walled polymer protective and insulating tube.

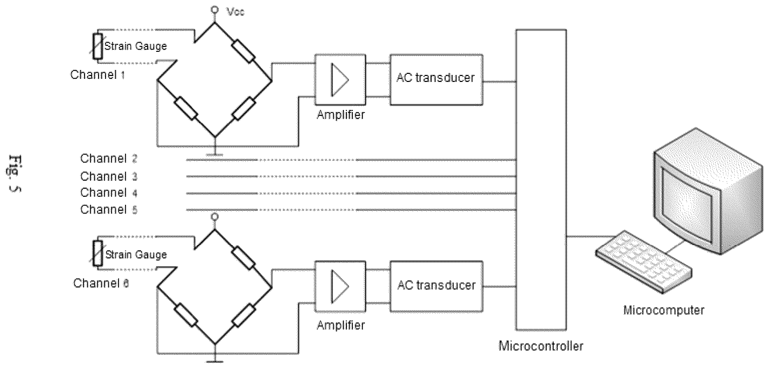

The microcontroller has several active channels, for example in fig. 5 it is 6 channels, enabling simultaneous collection of signals from all measuring systems of device 1 and receives at least one additional supplementary signal from other diagnostic equipment. The collected signals are then transmitted to an external device, for example to a receiving and recording station.

To ensure safe insertion of device 1 into the left ventricle and its operation inside this ventricle, device 1 must be made, at least in part, of a flexible material that allows it to be folded and placed within the catheter and then advanced within the transport catheter and unfolded after being withdrawn from the catheter. In other words, device 1 must be made of a material characterized by a very wide range of elastic deformations, high strength parameters and fatigue resistance, a relatively low Young's modulus and a narrow or zero range of plastic deformations. In addition, device 1 must be made of a material that is safe for use in living organisms and allows for visualization in imaging techniques.

The core 4 of the device 1 for measuring and recording the squeezing force according to the exemplary embodiment is rigid and made of surgical steel meeting the PN-EN ISO 9626 standard, while the flexible measuring arms 3 are made of a material that allows them to be bent under the influence of contraction and unbent back to a resting position during diastole of the left ventricle, without disturbing the natural function of the left ventricle, wherein the measuring arm 3 is made of a material and/or includes at least one visualization tag, i.e. a tag that allows the device 1 to be observed using imaging techniques such as X-ray or ultrasound to enable precise insertion and positioning in the left ventricle. More specifically, in one embodiment, the arms 3 are made of nitinol tape (Ni-Ti alloy, Kellog's Lab Research, USA). The sleeves 8 fixing the arms 3 and the sleeve 7 fixing the guide 6 are made of any plastic, medically acceptable alloy, e.g. silver. Stabilizer 5 is made of the same nitinol tape as arms 3. And both wires of the guide 6 are made of surgical hydrophilic wire, with the guiding wire having a diameter of 0.90 mm and the stabilizing wire having a diameter of 0.48 to 0.90 mm.

As mentioned, the device 1 in its folded form is introduced into the body inside, for example, a transport catheter of up to 6 mm in size and is introduced through the carotid artery. In its folded form, the device 1 has all arms 3 adjacent to the stabilization system 2, and upon retraction of the catheter, the arms 3 unfold to their target position - unfolded as shown in figs. 1a and 1b. During the measurement and recording of the squeezing force of the heart ventricle, the device 1 is located in the left ventricle in the target position and the wires emerging from the top of the core 4 are led through the carotid artery outside the patient to the microcontroller and the receiving-recording station. After completing the measurement, the arms 3 and the elements of the stabilizing system 2 are folded and hidden in the catheter pushed onto them, and then the device 1 is removed from the heart ventricle to the outside.

Device 1 placed in the left ventricle can be used to determine the value of the squeezing force generated by the wall of this ventricle during contraction. Since the strain gauges 9 placed and attached by gluing to the flexible measuring arms 3 deform in exactly the same way and to the same extent as the arms 3 on which they are mounted, the device 1 according to the invention enables the measurement of even the smallest changes in resistance, which are then recorded by means of a recording station, which makes it possible to determine the deformations of the arms 3 and to determine the squeezing force acting on the heart ventricle and, subsequently, to monitor the heart function.

According to the invention, there is also provided a computer-implemented method of measuring and recording the squeezing force measured using the device 1 in which, in the absence of a squeezing force, each strain gauge sensor 9 remains in the rest position and its measuring system indicates a voltmeter value of 0, whereas in the presence of a squeezing force, each strain gauge sensor 9 is deformed, changes its length as in fig. 4b and then the resistance value of the strain gauge changes under the influence of the deformation, which causes the measuring system of a given sensor 9 to indicate a voltmeter value other than 0, wherein this value is proportional to the resistance R of the sensor 9, which allows the squeezing force exerted by a specific area of the heart ventricular wall with which the measuring arm 3 of the device 1 is in contact during systole and diastole to be determined by converting the recorded voltage to the value of the squeezing force in accordance with the formula $F = (\varepsilon * E)/S$, as described above. In the invention, each flexible measuring arm 3 indicates one point value of the squeezing force exerted by the heart ventricle wall on its interior.

During operation of the device 1, all signals of changes in electric voltage (various values of voltmeter readings) caused by deformation of arms 3 are collected from strain gauges 9 of the device 1 and suitably amplified in voltage amplifiers integrated with analog-to-digital converters, after which, after amplification and processing, they are sent to a microcontroller, where they are recorded, and after completion, they are sent from this microcontroller via an asynchronous serial link to external equipment, i.e. any suitable equipment intended for capturing, storing and visualizing data, for example a microcomputer with the LINUX operating system. This microcomputer contains a computer-implemented program dedicated to reading data collected from device 1, enabling measurement and recording of the squeezing force and further visualization of the collected measurements and monitoring of the heart rate - the measurements are, after appropriate processing, presented in the form of graphical courses of changes in values as a function of time and then, using the above formulas, presented as the value of the squeezing force. Measurements can also be saved on this external hardware. The digital values of the graphs can also be saved in a spreadsheet-readable table for more precise analysis.

The above-mentioned embodiments are given herein only as non-limiting indications of the invention and cannot in any way limit the scope of protection, which is defined by the patent claims. It should be understood that any technical solutions used in the device and/or method according to the invention may be implemented using equivalent technologies without exceeding the scope of protection. The terms proximal, distal, upper and lower are merely for clarification purposes to facilitate understanding of the spatial arrangement of the invention and do not in any way limit the scope of protection set forth in the claims.

## Claims

1. A device (1) for measuring and recording the squeezing force exerted by the inner surface of the heart ventricle wall, **characterized in that** the device (1) comprises a stabilizing system (2) with at least one flexible measuring arm (3) extending therefrom, wherein the proximal end of the measuring arm (3) is connected with the stabilizing system (2) and the distal end rests on the endocardium of the left ventricle wall, wherein the device (1) has a spatial structure adapted to the interior of the left ventricle, allowing for multi-point contact with the endocardium surface, wherein the flexible measuring arm (3) deforms with contraction and diastole of the ventricle and is provided with a sensor (9) for measuring the deformation of the measuring arm (3), reflecting the squeezing force of the heart wall on the interior of the ventricle.

2. A device (1) for measuring and recording the squeezing force of the heart ventricle according to claim 1, **characterized in that** it comprises a stabilizing system (2) for maintaining a stable position of the device in the left ventricle, wherein the stabilizing system (2) comprises a stabilizer (5) and a core (4) running centrally in the long axis of the left ventricle with one end in the apex of the heart and the other end in the region of the aortic valve.

3. A device (1) for measuring and recording the squeezing force of the heart ventricle according to any one of claims 1 or 2, **characterized in that** the sensor (9) is a strain gauge sensor.

4. A device (1) for measuring and recording the squeezing force of the heart ventricle according to any one of claims 1-3, **characterized in that** the flexible measuring arms (3) attached to the core (4) of the stabilizing system (2) are arranged radially in relation to the long axis of the left heart ventricle, at equal angular distances from each other, on at least two levels, wherein the lengths of the measuring arms (3) are adjusted to the level and ensure adhesion to the periapical and middle segments of the left heart ventricle.

5. A device (1) for measuring and recording the squeezing force of the heart ventricle according to any one of claims 1-4, **characterized in that** the point of contact of the flexible measuring arm (3) with the endocardium is atraumatic, ensuring deformation of the measuring arm (3) during contraction, while at the same time without the risk of damaging the endocardium.

6. A device (1) for measuring and recording the squeezing force of the heart ventricle according to any one of claims 1-5, **characterized in that** the measuring arm (3) ends with one of the following: a loop, a ball, a spherical mesh.

7. A device (1) for measuring and recording the squeezing force of the heart ventricle according to any one of claims 1-6, **characterized in that** the sensor (9) placed on the measuring arm (3) is located as close as possible to the proximal end of the measuring arm (3).

8. A device (1) for measuring and recording the squeezing force of the heart ventricle according to any one of claims 1-7, **characterized in that** the flexible measuring arm (3) is made of a material that allows it to bend under the influence of contraction and to unbend it to a resting position during diastole of the left ventricle, without disturbing the natural function of the left ventricle, wherein the measuring arm (3) is made of a material and/or comprises at least one tag enabling its visualization.

9. A device (1) for measuring and recording the squeezing force of the heart ventricle according to any one of claims 1-8, **characterized in that** each flexible measuring arm (3) has dimensions allowing for the installation of a sensor (9) thereon.

10. A device (1) for measuring and recording the squeezing force of the heart ventricle according to any one of claims 1-9, **characterized in that** the stabilizing system (2) also comprises a stabilizing guide (6) ending with a curve in the apex of the heart, preferably the curve ending the stabilizing guide (6) is J-shaped, and the stabilizer (5) of the stabilizing system (2) is an O-shaped loop.

**11.** A device (1) for measuring and recording the squeezing force of the heart ventricle according to any one of claims 1-10, **characterized in that** the sensor (9) is connected to its own resistor system forming a Wheatstone bridge, which together with a voltmeter forms a measuring system generating an electric signal corresponding to the deflection force of the measuring arm (3), wherein the measuring system is connected to a microcontroller and the data collected by the sensors (9) are transmitted to the microcontroller.

**12.** A device (1) for measuring and recording the squeezing force of the heart ventricle according to any one of claims 1-11, **characterized in that** along the core (4) located centrally in the long axis of the left heart ventricle there are placed elements transmitting the measurement signal from the individual measurement systems to the microcontroller.

**13.** A device (1) for measuring and recording the squeezing force of the heart ventricle according to any of claims 11 or 12, **characterized in that** the microcontroller has a number of active channels enabling simultaneous collection of a signal from all measuring systems of the device (1), and receives at least one additional supplementary signal from other diagnostic equipment.

**14.** A device (1) for measuring and recording the squeezing force of the heart ventricle according to any one of claims 1-13, **characterized in that** each flexible measuring arm (3) indicates one point value of the squeezing force exerted by the wall of the heart ventricle on its interior.

**15.** A device (1) for measuring and recording the squeezing force of the heart ventricle according to any of claims 1-14, **characterized in that** the flexible measuring arms (3) and the elements of the stabilizing system (2) are folded and fit into a catheter that is inserted into the heart, wherein the measuring arms (3) and the elements of the stabilizing system (2) assume the measuring position after the catheter is slid off them in the left ventricle, then the distal ends of the measuring arms (3) rest on the endocardium of the left ventricle wall, and after the measurement is completed, the arms (3) and the elements of the stabilizing system (2) are folded and hidden in the catheter that is slid onto them and then removed from the heart ventricle to the outside.

**16.** A computer-implemented method of measuring and recording the squeezing force of a heart ventricle by measuring the squeezing force using a device for measuring and recording the squeezing force of a heart ventricle according to any one of claims 1-16, **characterized in that** in the absence of squeezing force, the sensor remains in a rest position and its measuring system indicates a voltmeter value of 0; in the presence of squeezing force, the sensor is deformed and its measuring system indicates a voltmeter value other than 0, wherein this value is proportional to the resistance R of the sensor, which allows determining the squeezing force exerted by a specific area of the heart ventricle wall with which the measuring arm of the device contacts during contraction and diastole, by converting the recorded voltage to a value of the squeezing force.

Fig. 1

a

b

c

Fig. 2a

Fig. 2b

Fig. 3

a)

b)

Fig. 4

Fig. 5

| | Europäisches Patentamt European Patent Office Office européen des brevets | **EUROPEAN SEARCH REPORT** | Application Number EP 24 46 1652 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/263933 A1 (KIGHT ALI [US] ET AL) 8 August 2024 (2024-08-08) * figures 1-10 * * paragraph [0003] - paragraph [0060] * * claims 1-11 * ----- | 1-16 | INV. A61B5/0215 A61B5/00 ADD. A61B5/021 |
| X | US 2008/255629 A1 (JENSON MARC [US] ET AL) 16 October 2008 (2008-10-16) * figures 11-12 * * paragraph [0150] - paragraph [0152] * * the whole document * ----- | 1-16 | |

TECHNICAL FIELDS SEARCHED (IPC)

A61B

The present search report has been drawn up for all claims

| Place of search | Date of completion of the search | Examiner |
|---|---|---|
| The Hague | 31 March 2025 | Knoop, Jan |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 24 46 1652

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2025

| Patent document cited in search report | Publication date | Patent family member(s) | | Publication date |
|---|---|---|---|---|
| US 2024263933 A1 | 08-08-2024 | NONE | | |
| US 2008255629 A1 | 16-10-2008 | US | 2008255629 A1 | 16-10-2008 |
| | | WO | 2006050385 A2 | 11-05-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0473070 B1
- US 6600948 B2